# EUROPEAN PATENT SPECIFICATION

(11) **EP 1 891 104 B1**
(45) Date of publication and mention of the grant of the patent: **26.08.2009**
(21) Application number: 06742805.2
(22) Date of filing: 04.05.2006
(51) Int. Cl.: C07K 1/04, C07K 14/575, C07K 17/08

(54) **SOLID PHASE BOUND THYMOSIN ALPHA1 AND ITS SYNTHESIS**
FESTPHASENGEBUNDENES THYMOSIN-ALPHA1 UND DESSEN SYNTHESE
THYMOSINE ALPHA1 LIEE EN PHASE SOLIDE ET SA SYNTHESE

(30) Priority: 04.05.2005 EP 05009758; 18.07.2005 US 699851 P
(43) Date of publication of application: 27.02.2008
(73) Proprietor: Lonza AG, 4052 Basel (CH)
(72) Inventor: ALBERICIO, Fernando, 08007 Barcelona (ES); CRUZ, Luis, Javier, 08028 Barcelona (ES); GARCIA, Ramos Yésica, 08917 Barcelona (ES); TULLA-PUCHE, Judit, 08028 Barcelona (ES)
(86) International application number: PCT/EP2006/004192
(87) International publication number: WO 2006/117227

(56) References cited:
- DATABASE CA [Online] CHEMICAL ABSTRACTS SERVICE, COLUMBUS, OHIO, US; HAN, XIANG ET AL: "Study on Fmoc solid-phase synthesis and activity of thymosin alpha1" XP002391161 retrieved from STN Database accession no. 2005:987404 & ZHONGGUO YAOWU HUAXUE ZAZHI , 14(1), 27-29 CODEN: ZYHZEF; ISSN: 1005-0108, 2004,
- VIRTA P ET AL: "Solid-supported synthesis of oligomeric bioconjugates" TETRAHEDRON, ELSEVIER SCIENCE PUBLISHERS, AMSTERDAM, NL, vol. 59, no. 28, 7 July 2003 (2003-07-07), pages 5137-5174, XP004434882 ISSN: 0040-4020

## Description

The present invention relates to a method of solid-phase peptide synthesis for a peptide that is unusually difficult to synthesize, and to respective peptide-solid phase conjugates.

The challenge of solid-phase peptide synthesis is that despite well-established routine methods exist, successful and efficient synthesis is highly dependent on the very peptide sequence to be synthesized.

Some peptide sequences, drawn from natural peptide or protein segments, simply do much worse than others on the same resins materials used highly succesfully with a lot of 'normal' peptides. In the literature, often careful fine-tuning of solvent systems has been proposed in view of preventing inter-chain aggregation on the resin by virtue of beta-sheet formation. Still then, solvent engineering using dichloromethane, N-methylpyrrolidone or dimethylformamide, eventually supplemented with helical structure-stabilising particular polar solvents such trifluoroethanol, is far from providing a general remedy for difficult peptides.

Thymosin α₁ (international non-proprietary pharmaceutical name: thymalfasin) is an N-acetylated 28-mer peptide hormone that occurs in the human thymus gland and is used a drug to treat chronic hepatitis B.

Its linear full-length solid-phase synthesis has been described early on but suffered from poor yields up to now. Unfortunately, the Thymosin α₁ amino acid sequence does not offer a glycine or proline which could be used as a junctional residue in a classical segment coupling approach in a stepwise approach to synthesis.

Echner et al. (Liebigs Ann. Chem. 1988, 1095-1097) describe linear solid-phase synthesis on a Wang-polystyrene resin. Whilst coupling efficiency is allegedly checked after each coupling with BOP reagent in DMF by means of Kaiser Test, only after every fifth coupling capping of uncoupled peptide by means of acetylation is done. Cleavage of the protected and terminally acetylated peptide from the resin is achieved with concentrated trifuoro acetic acid; thereafter, resin and liquid phase are separated by filtration and the peptide product is collected by precipitation with dieethylether. The precipitate collected in this way is quantiated and is designated 'crude product', yield is calculated as to amount of 76%. - Only thereafter, a first chromatographic purification of the crude product is undertaken, followed by reverse phase HPLC. No yield is indicated for the pure product thus obtained.

A problem with Echner's approach is that incomplete chain coupling, leading to shortened undesirable sequence variants during synthesis, is not accounted for in the yield calculation. 'Crude product' amounts to every peptidic species obtained during synthesis, including incompletely deprotected/alkylated peptide. However, purity peptide obtained from linear synthesis is problem when dealing with difficult peptides; interestingly, Echner does not give any indication of yield of purified product thus obtained, nor is any HPLC chromatogram given in the publication as is routinely done in the art for proving quality of synthesis. In our hands, the synthetic scheme of Echner gives only very low yields of correct product, a large array of improper peptide product making up for most of the crude product.

Another problem with Echner's approach which the author raises himself (p.1095, left column, 3^{rd} para) is loading of the Wang resin with the C-terminal amino acid of Thymosin αᵢ which is an asparagine; the loading Echner achieves is 0.15 mmol/g, which is extremely low. Fmoc-Asn(4,4'-dimethoxydiphenylmethyl-)-OH amino acid must be used for loading of the resin without running risk of degrading side-reactions. The publication by Xiang Han (Zhonggen Yaoweter thaxue Zathi, 2009, 14(1), 27-29) discloses another Thymosim de synthesis. It is the object of the present invention to devise an improved solid-phase synthesis for Thymosin α₁. This object is solved by the method of claim 1.

According to the present invention, a method of synthesizing on a solid phase Thymosin α₁ or a C-terminally truncated version comprising residues 1-27 or an N-terminally truncated version comprising at least residues 19-28 or an truncated version comprising at least residues 19-27 of mature Thymosin α₁ is devised, comprising the steps of
a. coupling a suitably protected FMOC-Glu or FMOC-Asn residue onto a PEG resin which PEG resin is preferably selected from the group comprising polystyrene-PEG mixed resins, PEG polyether-polyester mixed resins or PEG polyether-polyamide mixed resins and essentially pure PEG resins
b. elongating the peptide chain by FMOC synthesis wherein the side-chains of individual amino acids may be derivatized with suitable protecting groups
c. optionally acetylating the last, N-terminal residue and
d. cleaving the peptide from the resin

The present method allows of synthesizing the thymalfasin peptide in unprecedented purity and hence high yield in a single solid-phase linear synthetical approach. According to the present invention, it may also be feasible though to synthesize only a C-terminal fragment on a solid phase and to constitute any full-length peptide or an at least longer version of the peptide by segment coupling, using in one preferred embodiment coupling of an N-terminal fragment having a C-terminal Ser or Thr which is protected to racemization by the pseudoproline dipeptide method (Wöhr et al., J. Am. Chem. Soc. 118, 9218).

Preferably, the peptide to be synthesized is Thymosin α₁ (also refered to as mature, active Thymosin α₁ as to distinguish from precursor peptides) having the sequence
1-Ser-Asp-Ala-Ala-Val-Asp-Thr-Ser-Ser-Glu-Ile-Thr-Thr-Lys-Asp-Leu-Lys-Glu-Lys-Lys-Glu-Val-Val-Glu-Glu-Ala-Glu-Asn-28
   wherein the individual amino acid chains are unprotected or are suitably protected, as need may be for a given individual amino acid residue as is common knowledge in the art (see Bodansky, infra, for instance). More preferably, such peptide is N-terminally acetylated in a step subsequent to linear synthesis but prior to cleavage from resin. Natural tymalfasin which is also used as a pharmaceutical is N-terminally acetylated.

Notably, the present inventors have found that it is mainly the C-terminal half of the thymalfasin peptide, it is about residues 19-28, that incur most of the impurities by chain deletions and hence losses in final product yield generated during linear phase synthesis. This part of the peptide has been found to be extremely difficult to synthesize.

Vice versa, during linear synthesis, it is also feasible to use such oxazolidine dipeptides according to Wöhr et al., supra. A benefit of using such dipeptide instead of individual protected, preferably Fmoc-, amino acid can be inferred from the tymalfasin sequence in particular for the dipeptide Asp-Thr; use of oxazolidine dipeptide derivative would help here to avoid aspartimide formation during synthesis.

Similiarly, prevention of glutarimidc formation may rcquirc use of special protecting groups.

Whilst the present invention strongly prefers Fmoc chemistry for carrying out the coupling reactions, Boc chemistry may likewise be used in another embodiment to carry out the present invention.

Coupling reagents for peptide synthesis are well-known in the art (see Bodanszky, M., Principles of Peptide Synthesis, 2nd ed. Springer Verlag Berlin/Heidelberg, 1993; also see discussion of role of coupling additives or auxilliaries therein). Coupling reagents may be mixed anhydrides (e.g. T3P: propanephosphonic anhydride) or other acylating agents such as activated esters or acid halogenides (e.g. ICBF, isobutylchloroformate), or they may be carbodiimides (e.g. 1-ethyl-3-(3-dimethylaminopropyl)-carbodiimide), activated benzotriazine-derivatives (DEPBT: 3-(diethoxyphosphoryloxy)-1,2,3-benzotriazine-4(3H)-one) or uronium or phosphonium salt derivatives of benzotriazole.

In view of best yield, short reaction time and protection against racemization during chain elongation, more preferred is that the coupling reagent is selected from the group consisting of uronium salts and phosphonium salts of the benzotriazole capable of activating a free carboxylic acid function along with that the reaction is carried out in the presence of a base. Suitable and likewise preferred examples of such uronium or phosphonium coupling salts are e.g. HBTU (O-(1H-benzotriazol-1-yl)-N,N,N',N'-tetramethyluronium hexafluorophosphate), BOP (benzotriazol-1-yloxy-tris(dimethylamino)phosphonium hexafluorophosphate), PyBOP (Benzotriazol-1-yloxy-tripyrrolidinophosphonium hexafluorophosphate), PyAOP, HCTU (O-(1H-6-chlorobenzotriazol-1-yl)-1,1,3,3- tetramethyluronium hexafluorophosphate), TCTU (O-(1H-6-chlorobenzotriazol-1-yl)-1,1,3,3-tetramethyluronium tetrafluoroborate), HATU (O-(7-azabenzotriazol-1-yl)-1,1,3,3-tetramethyluronium hexafluorophosphate), TATU (O-(7-azabenzotriazol-1-yl)-1,1,3,3-tetramethyluronium tetrafluoroborate), TOTU (O-[cyano(ethoxycarbonyl)methyleneamino]-N,N,N',N'-tetramethyluronium tetrafluoroborate), HAPyU (O-(benzotriazol-1-yl)oxydipyrrolidinouronium hexafluorophosphate.

Preferably, when using DEPBT or the like, uronium or phosphonium salt reagents, a further or second weak base reagent is needed for carrying out the coupling step. This is matched by base whose conjugated acid has a pKa value of from pKa 7.5 to 15, more preferably of from pKa 7.5 to 10, with the exclusion of an α-amino function of a peptide or amino acid or amino acid derivative, and which base preferably is a tertiary, sterically hindered amine. Examples of such and further preferred are Hünigbase ( N,N-diisopropylethylamine), N,N-dialkylaniline, 2,4,6-trialkylpyridine or N-alkyl/morpholine with the alkyl being straight or branched C1-C4 alkyl, more preferably it is N-methylmorpholine or collidine (2,4,6-trimethylpyridine), most preferably it is collidine.

The use of coupling additives, in particular of coupling additives of the benzotriazole type, is also known (see Bodanszky, supra). Their use is particularly preferred when using the highly activating, afore said uronium or phosphonium salt coupling reagents. Hence it is further preferred that the coupling reagent additive is a nucleophilic hydroxy compound capable of forming activated esters, more preferably having an acidic, nucleophilic N-hydroxy function
wherein N is imide or is N-acyl or N-aryl substituted triazeno, most preferably the coupling additive is a N-hydroxy-benzotriazol derivative (or 1-hydroxy-benzotriazol derivative) or is an N-hydroxy-benzotriazine derivative. Such coupling additive N-hydroxy compounds have been described in large and wide in WO 94/07910 and EP-410 182. Examples are e.g. N-hydroxysuccinimide, N-hydroxy-3,4-dihydro-4-oxo-1,2,3-benzotriazine (HOOBt), 1-hydroxy-7-azabenzotriazole (HOAt) and N-hydroxybenzotriazole (HOBt). N-hydroxybenzotriazine derivatives, first and foremost HOOBt, are particularly preferred.
Ammonium salt compounds of coupling additives are known and their use in coupling chemistry has been described, for instance in US 4806641.

In a further particularly preferred embodiment, the uronium or phosphonium salt coupling reagent is an uronium salt reagent, preferably it is HCTU, TCTU or HBTU, more preferably it is HCTU or TCTU, and most preferably it is used in the reaction in combination with N-hydroxy-3,4-dihydro-4-oxo-1,2,3-benzotriazine or a salt thereof. In the context of the present invention, it is to be noted that HCTU and TCTU are defined as to be encompassed by the term 'uronium salt reagent' despite that these compounds and possible analogues have been shown to comprise an isonitroso moiety rather than an uronium moiety by means of crystal structure analysis (O. Marder, Y. Shvo, and F. Albericio "HCTU and TCTU: New Coupling Reagents: Development and Industrial Applications", Presentation Gordon Conference Feb. 2002 & Chimica Oggi 2002, 20:37-41), an N-amidino substituent on the heterocyclic core giving rise to a guanidium structure instead. In the present context, such class of compounds is termed 'guanidium-type subclass' of uronium salt reagents according to the present invention.

Deprotection of the base labile Nα may be carried out as routinely done in the art, e.g. with 20% piperidine in N-methylmorpholine in case of Fmoc chemistry. The last amino acid to be added, e.g. typically the N-terminal Serine of mature tymalfasin, may of course carry an N-terminal protecting group other than Fmoc, e.g. it may carry Boc or readily an acetyl-protecting group, though the latter may more conveniently introduced by post-coupling acetylation of the deprotected N-terminal amino acid of the peptide usually. Preferably, one may use an orthogonal protecting group other than Fmoc for protection of the last, N-terminal residue. Examples are Alloc protecting groups (e.g. for protecting primary amines), both being selectively removable from peptide by Pd(0) catalyzed transacylation (Gomez-Martinez, Nα-Alloc temporary protection in solid-phase peptide synthesis, use of amine-borane complexes as allyl group scavengers, J. Chem. Soc. , Perkin Trans. 1, 1999, 2871-2874), the Dde group (Bycroft et al., A novel lysine protecting procedure for SPPS of branched peptides, J. Chem. Soc. Chem. Commun. 1993, p. 778-779), a dimedon derivative that is removable by hydrazinolysis, and its functional homologues such as e.g. Nde group (N-1-(4-nitro-1,3-dioxoindan-2-ylidene)-ethyl group, Kellam et al., Tetrahedron 54, 1998, p. 6817-6832; N-1-(4,4-dimethyl-2,6-dioxocyclohexylidene)-3-methylbutyl, Chan 1995). Dde, its derivatives and homologues are collectively addressed as Dde-type protecting groups sharing in their free, unreacted state a functional dioxoalkylidene moiety of formula III

III (-CO-)₂C=C(-R)(-OH)

wherein R is substituted or unsubstituted alkyl and wherein preferably the two carbonyl functions are forming a cyclic structure connected by a -CH2-CR'R"-CH2- , -NR'-CO-NR"-or-CR'=CR"- backbone. Preferably, R',R" are then alkyl or, taken together, aryl.

It is also readily feasible to use modified or tagged orthogonal protecting groups, which bear the additional benefit of allowing of simple purification by subsequent affinity chromatography or affinity-improved 'standard' RP HPLC using such reversible affinity tag. In this case, the peptide synthesized is cleaved off the resin suitably under mild condition avoiding global deprotection, purification is taking place, the N-terminal probe is selectively removed allowing of subsequent N-terminal acetylation and finally global deprotection of peptide . An example of such are the Fmoc derivatives described in Ball et al., Int. J. Peptide Protein Res. 1992, 40:370-379 and Ball et al., J. Chromatography 1994, 686:73-88.

Particularly preferred for the present invention is to carry out removal of the Fmoc group with 20% piperidine-DMF (v/v), e.g. (2 × 1 min, 2 × 10 min, 1 × 5 min). Likewise preferred, couplings of Fmoc-aa-OH (5 equiv) are carried out with the above mentioned coupling reagents in DMF or similar solvent for 60-120 min. For optimizing synthesis and in particular coupling times for individual sequence positions, after the coupling ninhydrin tests were carried out and if it was positive the coupling was repeated under the same conditions, otherwise the process was continued. If after second coupling the ninhydrin test was still positive an acetylation step was carried out with Ac₂O and DIEA or similar method for capping the deletion peptide chains. Such enhanced coupling and/or capping procedure was then applied to repeated synthesis under the same conditions for the very given sequence position. Notably, choice of resin was found to strongly influence sequence-position specific coupling problems as is exemplified in the experimental section.

According to the present invention, the solid-phase comprises a PEG resin. According to the present inventions, they allow of synthesizing tymalfasin or the difficult core peptide section of tymalfasin in exceptional yield and purity. In the present context, the definition of 'solid-phase' comprises apart from the inert resin matrix the presence of an integral linker on the inert matrix material, for linkage of peptide or for linking a further, grafted linker or handle.

Such resins have been described e.g. in US 2003078372 A1; by virtue of the PEG incorporated into the solid-phase, such resins gain amphiphilic properties which makes them different from traditional resin materials. The are known to adopt a 'gel-like' behaviour instead of the solid-like behaviour of traditional e.g. Merrifield resin after swelling. This particular feature is said to offer benefits of reduced cycling times in view of diffusion/liquid exchange e.g. during washing, coupling, deprotection steps etc. Notably, according to the present invention, such PEG resin is also found to improve efficiency of difficult, peptide-specific coupling steps for a peptide, drastically and in an unexpected, synergistic fashion enhancing purity and yield. The term '*PEG resin*' is to be construed strictly *generically* in the present context as to relate to a polymeric resin comprising a polyether copolymeric or block copolymer share at least - a PEG-type polyether or PEG-polyether resin for short. The term polyether as used here relates in its conventional sense to polyoxyalkenyl polymers such as e.g. polyoxyethylene or polyethyleneglycol (PEG) respectively, polyoxypropylene, polyoxybutylene or mixed polymers thereof. Different embodiments of such type of resin exist, polymerisation of carbon monomers (including monomer units other than oxirane) not being restricted to polyether chemistry only. Accordingly, the resin matrix of the solid-phase may be constituted e.g. by an amphipilic polystyrene-PEG mixed resin (e.g. Tentagel, see US 4908405) or PEG-polyamide or PEG-polyester mixed resin, e.g. Kempe et al., J. Am. Chem. Soc. 1996, 118, 7083; also cp.
US 5,910,554 A. Usually, resin loading is usually less efficient and/or chemical stability in particular in acidic media is often not comparable to that of traditional resins, e.g. classic Merrifield polystyrene-DVH. Polystyrene-PEG mixed resins are reaching higher loadings, but lower amphiphilicity is obtained because the PEG content is decreased.

It is also possible to use a PEG resin that has been obtained by grafting of a non-PEG resin matrix with a layer of a suitable PEG resin material, e.g. cp. Kates et al., High-load polyethylene glycol-polystyrene (PEG-PS) graft supports for solid-phase synthesis, Biopolymers 1998, 47:365-380.

In a strongly preferred embodiment according to the present invention, the PEG resin is a substantially pure PEG-polyether resin that is more preferably devoid of any ,eventually further substituted, polystyrene co-polymeric or block-copolymeric share and, again more preferably, further does also not comprise any internal ester or amide functional groups but only polyether functional groups. A substantially pure PEG-polyether resin may also be termed an essentially pure PEG-polyether resin or a resin consisting essentially of PEG-polyether share as defined above. Most preferably, it is a pure PEG resin or PEG-polyether resin. Preferably, the meaning of 'substantially pure' is that the above structural definition of pure PEG resin amounts to at least 70% of the dry weight of the solid-phase material, allowing of a minor share of polymeric additions of other type. Such ideally pure PEG resin material offers optimal chemical stability along with good compatibility and ease of handling with standard solvents during normal Fmoc synthesis. Notably, in the latter definition the terminal ester or amide bonds bridging the resin directly or via the integral and/or grafted linker to the peptide are not accounted for, since they are not internal structural, crosslinking features and accordingly do not affect the definition of 'pure PEG' in this regard. Such 'pure', highly amphiphic PEG resins are offered by different companies, e.g. Matrix Innovations Inc. from Quebec, Canada ('ChemMatrix' brand) or Versamatrix A.S. from Denmark and are described e.g. in WO 02/40559. They may offer loading capacity of more than 0.5 mmol/g, see ChemMatrix brand resin described in afore said WO'559 for instance. For linkage of peptide, such resin may have terminal integral linkers such as hydroxymethyl radicals or derived thereof 'quasi-native' aminomethyl, carboxyl or bromomethyl or iodomethyl radicals for instance, or may be derivatized by known grafted linkers or handles for solid phase linkage such as e.g. Wang, PAL, 4-alkoxybenzaldehyde or Rink or Sieber amide linkers.

Notably, as a further element of the surprising properites of PEG resin according to the present invention, coupling of the second amino acid No. 27, Glu, was suddenly found to become a critically difficult step, in particular when using side-chain anchoring of the terminal Asp/Asn residue as compared to traditional resins (e.g. PS-DVB or CTC-PS-DVB), whilst the coupling efficiency of the other critical residues in the sequence stretch 19-28 was found to be improved, though not rendered trivial. Hence extended and/or repeated coupling of residue No. 27 to the resin-anchored Asn moiety was required when using PEG resin, and especially pure or substantially pure PEG-polyether resin. 'Extended' relates to using more equivalents of an Fmoc amino acid or longer coupling times than for the average residue.

Further objects of the present invention are the various peptide-solid phase conjugates obtained in this way. The definitions and explanations given in the foregoing apply likewise to these objects.

Such object is a peptide conjugate of formula I 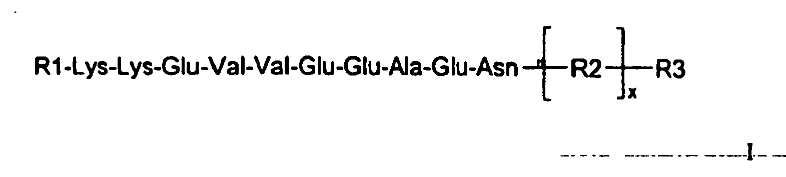 wherein each amino acid residue may be individually protected or unprotected, R3 is a PEG-resin solid phase which is comprising an integral linker, R2 is a grafted linker with x being 0 or 1 and R1 is hydrogen, a protecting group or a peptidyl radical, preferably a peptidyl radical counting less than 50, preferably less than 25 amino acid residues, and wherein, if R1 is a peptidyl radical, the individual amino acid residues of said radical are individually protected or unprotected and the N-terminus of said peptidyl radical is free, is acetylated or is protected with a protecting group Y.

Accordingly, preferably R1 is a peptidyl radical which is Y-Ser-Asp-Ala-Ala-Val-Asp-Thr-Ser-Ser-Glu-Ile-Thr-Thr-Lys-Asp- Leu-Lys-Glu-, Ac-Ser-Asp-Ala-Ala-Val-Asp-Thr-Ser-Ser-Glu-Ile-Thr-Thr-Lys-Asp- Leu-Lys-Glu or H- Ser-Asp-Ala-Ala-Val-Asp-Thr-Ser-Ser-Glu-Ile-Thr-Thr-Lys-Asp- Leu-Lys-Glu-Leu-Lys-Glu with Y having the meaning named before. Ac: Acetyl.

Linkers as defined by Guillier, Orain, and Bradley, Chem. Rev. 100, 2091-2157, 2000 are considered simply as immobilized protecting groups and are classified into one of two types: (a) *Integral linkers* in which part of the solid phase core material forms or is inseparably part of the linker, both together constituting R3, and (b) *Nonintegral or grafted linkers R2* in which the linker R2 is additionally attached to the solid support, on top of an integral linker/solid phase R3, e.g. for allowing of more mild cleavage conditions. Typical examples of integral linkersolid phases are p-methylbenzhydrylamine (MBHA), 2-chlorotrityl (CTC), aminomethyl, etc. Integral linkers are mandatory whilst grafted linkers R2 are optional. Typical examples are 4-hydroxymethylphenoxyacetyl- or 4-hydroxymethylbenzoic acid.

Conventionally, the peptide is conjugated through its C-terminal residue via its 1-carboxylic acid function to the solid phase or the grafted linker, respectively.

Preferably, according to the present invention, the synthesis of tymalfasin or of its afore mentioned fragments and variants is achieved by side chain anchoring of the second last-Glu or of a C-terminal Asp or Asn residue.'Second last-Glu' may mean, in the present context, a Glu-Asn protected dipeptide that is anchored to a solid phase or it may mean that synthesis of a variant of tymalfasin that is devoid of the C-terminal Asn of natural tymalfasin starts with said Glu residue. Unexpectedly, side chain anchoring has been found to result in more efficient synthesis. A terminal Asp residue that is anchored via its beta-carboxylic function to the solid phase or a grafted linker may be post-synthetically amidated, after cleavage from resin. More preferably though, an Fmoc-Asp which is suitably protected at its C-carboxylic function, preferably by means of a tert-butyl protecting group, is side-chain anchored to an amide generating linker, preferably a grafted amide-generating linker, with regard to the final product after cleavage from resin, such conjugate may be considered and termed a 'side-chain anchored Asn' as well due to the use of an amidegenerating linker. Examples and strongly preferred embodiments of such amide generating linkers are e.g. 'Rink amide' 4-(2',4'-dimethoxybenzyl-aminomethyl)-phenoxy- resin, Sieber resin (Tetrahedron Lett. 1987, 28, 2107-2110) or similiar 9-aminoxanthenyl-type resins, PAL resins (Albericio et al., 1987, Int. J. Pept. Protein Research 30, 206-216). Another, less preferred example for amide-generating linkers are the specially substituted trityl-amine derivatives according to Meisenbach et al., 1997, Chem. Letters , p. 1265 f.). These are examples of (Fmoc-chemistry compatible) linker groups from which a Ca-carboxamide is generated or liberated upon cleavage of the peptide from the resin. It goes without saying that use of such amide linkers is of course dependent on the type of solid phase synthesis carried out, i. e. whether is traditional Boc or now customary, orthogonal Fmoc protection chemistry that is used for coupling; a Boc-specific amide resin linker is PAM, for instance. Accordingly, solid phases comprising such linker groups are termed 'amide-producing solid phases' in the present context. Incorporation of Fmoc-Asp-OtBu onto a solid-phase, containing one or two linkers, using coupling reagents (Albericio, van Abel, Barany, Int. J. Peptide Protein Res., 35, 284-286, 1990), has been described and may be applied in the context of the present invention. Usually, uronium type coupling reagents are preferred for achieving this.

Advantages of this side-chain anchoring strategy are:
(i) Incorporation of the first amino acid into the solid support is carried out with rather quantitative yields through an amide bond and therefore the same reagents used for the formation of peptide bond can be used. In addition, the incorporation of asparagine derivatives into a Wang type resin takes place with low yields, with risk of racemization and with formation of β-cyanoalanine if side-chain amide is not protected (Katsoyannis et al., 1958, J. Am. Chem. Soc. 80:2558). Furthermore, side-chain unprotected asparagines could be incorporated to halogen resins through both the 1-carboxylic and the carboxamide function, requiring use of e.g. suitably protected Fmoc-Asn(Mbh)-OH amino acids, creating further problems at the stage of deprotecting the peptide finally.
(ii) Side-chain anchoring strategy involving just acid-labile tBu-based protecting groups (tBu esters for the 1-carboxylic acid of the first residue and ω-carboxylic acids of Asp and Glu residues, tBu ethers for Scr and Thr side-chain) and acid-labile Boc for Lys side-chain will facilitate the final global deprotection, because simple scavengers such as H₂O can be used. In contrast, if Asn is incorporated with trityl, xantyl, or 2,4,6-trimethoxybenzyl protecting groups, it will require the presence of additional scavengers.
(iii) The growing backbone peptide chain into the side-chain anchoring strategy will have more flexibility than in the 1-carboxylic anchoring strategy, because the anchoring is through the side-chain.

Scheme I below recapitulates the most preferred mode of synthesis on a PEG resin according to the present invention for tymalfasin (Zadaxine), most preferably on a pure or substantially pure PEG resin, wherein linker 1 is an amide-generating linker and linker 2 is an integral linker: 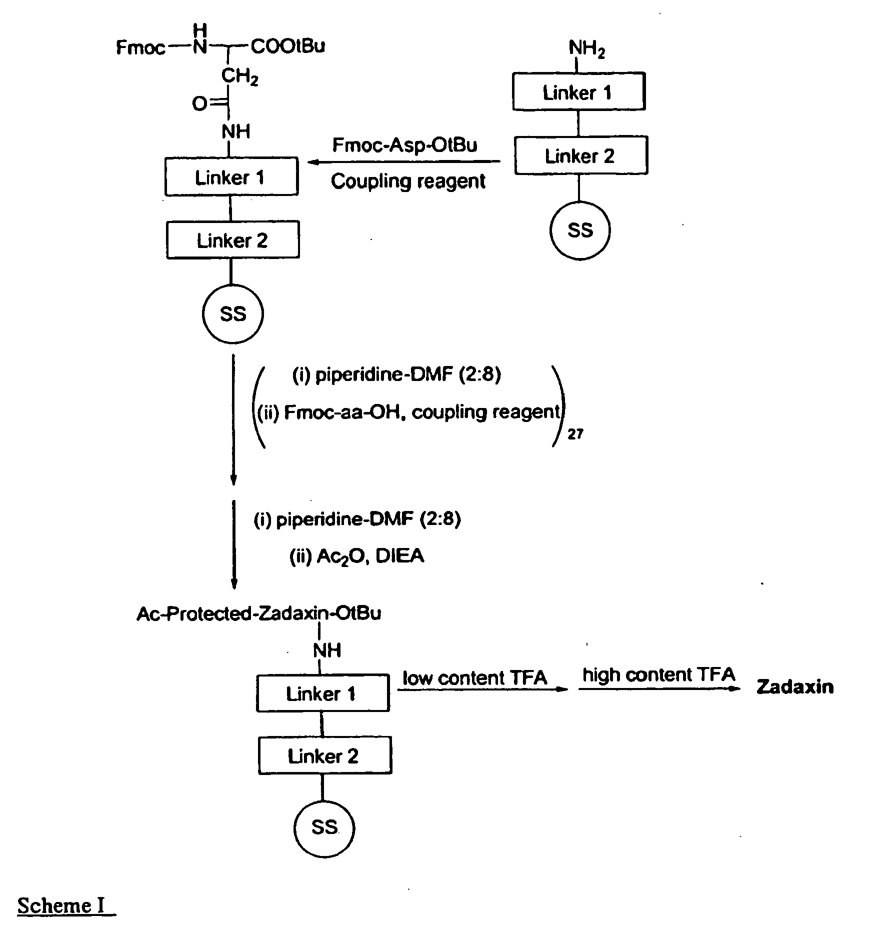
(i) Incorporation of Fmoc-Asp-OtBu onto a solid support (SS), containing one or two linkers, using coupling reagents (Albericio, van Abel, Barany, Int. J. Peptide Protein Res., 35, 284-286, 1990).
(ii) elongating the peptidic chain with the rest of Fmoc-protected amino acid (tBu for Asp, Glu, Ser, and Thr; and Boc for Lys).
(iii) acetylation
(iv) cleavage of the peptide from the solid support and removal of the protecting groups, which can be carried out in one (high acid concentration solution) or two steps (low acid concentration solution followed by high acid concentration solution).

### EXAMPLES

General Procedures. Fmoc-Sieber-PS-resin was from NovaBiochem (Läufelfingen, Switzerland), ChemMatrix resin from Matrix Innovation (Quebec, Canada), 2-Cl-TrtCl-resin from CBL (Patras, Greece), HCTU from Luxembourg Industries Ltd. (Tel Aviv, Israel), Protected Fmoc-amino acid derivatives from IRIS Biotech (Marktredwitz, Germany).
Solid-phase syntheses were carried out in either glass funnels fitted with filters or polypropylene syringes (50 mL) fitted with a polyethylene porous disc. Solvents and soluble reagents were removed by suction. Removal of the Fmoc group was carried out with piperidine-DMF (2:8, v/v) (2 × 1 min, 2 × 10 min, 1 x 5 min). Washings between deprotection, coupling, and, again, deprotection steps were carried out with DMF (5 × 0.5 min) and CH₂Cl₂ (5 × 0.5 min) using each time 10 mL solvent/g resin. Peptide synthesis transformations and washes were performed at 25 °C. Syntheses carried out on solid-phase were controlled by HPLC of the intermediates obtained after cleaving with TFA-H₂O (95:5) for 1 h an aliquot (aprox. 2 mg) of the peptidyl-resin. HPLC reversed phase columns Symmetryl^{™} C₁₈ 4,6 × 150 mm, 5 µm (column A) and were from Waters (Ireland). Analytical HPLC was carried out on a Waters instrument comprising two solvent delivery pumps (Waters 1525), and automatic injector (Waters 717 autosampler), dual wavelength detector (Waters 2487). UV detection was at 215 or 220 nm, and linear gradients of CH₃CN (+0.036% TFA) into H₂O (+0.045% TFA), from 30% to 100% in 15 min.
MALDI-TOF and ES-MS analysis of peptide samples were performed in a PerSeptive Biosystems Voyager DE RP, using ACH matrix.

### Example 1

### Ac-Ser(tBu)-Asp(OtBu)-Ala-Ala-Val-Asp(OtBu)-Thr(tBu)-Ser(tBu)-Ser(tBu)-Glu(OtBu)-Ile-Thr(tBu)-Thr(tBu)-Lys(Boc)-Asp(OtBu)-Leu-Lys(Boc)-Glu(OtBu)-Lys(Boc)-Lys(Boc)-Glu(OtBu)-Val-Val-Glu(OtBu)-Glu-(OtBu)-Ala-Glu(OtBu)-Asp(Rink-ChemMatrix-resin)-OtBu

### Step 1

### Side chain anchoring of Fmoc-Asp(Rink-ChemMatrix-resin)-OtBu

Fmoc-Rink-ChemMatrix-resin (0.45 mmol/g) (5 g, 2.25 mmol) was placed in a glass funnel fitted with a porous disk. The resin was subjected to the following washings/treatments with CH₂Cl₂ (3 × 0.5 min), DMF (3 × 0.5 min), and piperidine as indicated in General Procedures, and DMF (5 × 0.5 min). Then, Fmoc-Asp-OtBu (4.11 g, 5 equiv) and DIEA (3.4 mL, 10 equiv) in DMF (9 mL) were added, followed by HCTU (3.96 g, 4.8 equiv) in DMF (5 mL). The mixture was left to stir mechanically for 2 h and the resin was washed with DMF (3 × 0.5 min) and the ninhydrin test was negative. Ac₂O (0.5 mL, 5 mmol) and DIEA (1.7 mL, 5 mmol) in DMF (14 mL) were added and left to stir mechanically for 15 min, where the resin was filtered and washed with DMF (3 × 0.5 min). Successful loading was determined as to amount to 0.4 mmol/g.

### Step 2

### H-Ser(tBu)-Asp(OtBu)-Ala-Ala-Val-Asp(OtBu)-Thr(tBu)-Ser(tBu)-Ser(tBu)-Glu(OtBu)-Ile-Thr(tBu)-Thr(tBu)-Lys(Boc)-Asp(OtBu)-Leu-Lys(Boc)-Glu(OtBu)-Lys(Boc)-Lys(Boc)-Glu(OtBu)-Val-Val-Glu(OtBu)-Glu(OtBu)-Ala-Glu(OtBu)-Asp(Rink-ChemMatrix-resin)-OtBu

The Fmoc group was removed and Fmoc-aa-OHs (5-10 equiv) were sequentially added to the above peptidyl-resin (step 1) together with DIEA (3.4 mL, 10 equiv) in DMF (9 mL), by HCTU (3.96 g, 4.8 equiv) in DMF (5 mL). The mixture was left to stir mechanically for 1-2 h, the resin was washed with DMF (3 × 0.5 min) and the ninhydrin test was taken. If test resulted positive (blue), the coupling was repeated, while if the test was slightly (brown), an acetylation step was carried out with Ac₂O (0.5 mL, 5 mmol) and DIEA (1.7 mL, 5 mmol) in DMF (14 mL) for 15 min with mechanical stirring. Aliquots of resins were taken after piperidine treatments. At positions E27, K19, for each position a double coupling with at least 35 min. reaction time per single and using 10 eq. of Fmoc amino acids was performed.

### Step 3

### Ac-Ser(tBu)-Asp(OtBu)-Ala-Ala-Val-Asp(OtBu)-Thr(tBu)-Ser(tBu)-Ser(tBu)-Glu(OtBu)-Ile-Thr(tBu)-Thr(tBu)-Lys(Boc)-Asp(OtBu)-Leu-Lys(Boc)-Glu(OtBu)-Lys(Boc)-Lys(Boc)-Glu(OtBu)-Val-Val-Glu(OtBu)-Glu(OtBu)-Ala-Glu(OtBu)-Asp(Rink-ChemMairix-resin)-OtBu

The final acetylation was carried out with Ac₂O (0.5 mL, 5 mmol) and DIEA (1.7 mL, 5 mmol) in DMF (14 mL) for 15 min with mechanical stirring, where ninhydrin was negative. The resin was dried and 16.5 g were obtained.

### Step 4

### Ac-Ser-Asp-Ala-Ala-Val-Asp-Thr-Ser-Ser-Glu-Ile-Thr-Thr-Lys-Asp-Leu-Lys-Glu-Lys-Lys-Glu-Val-Val-Glu-Glu-Ala-Glu-Asn-OH (Zadaxin,tymalfasin)

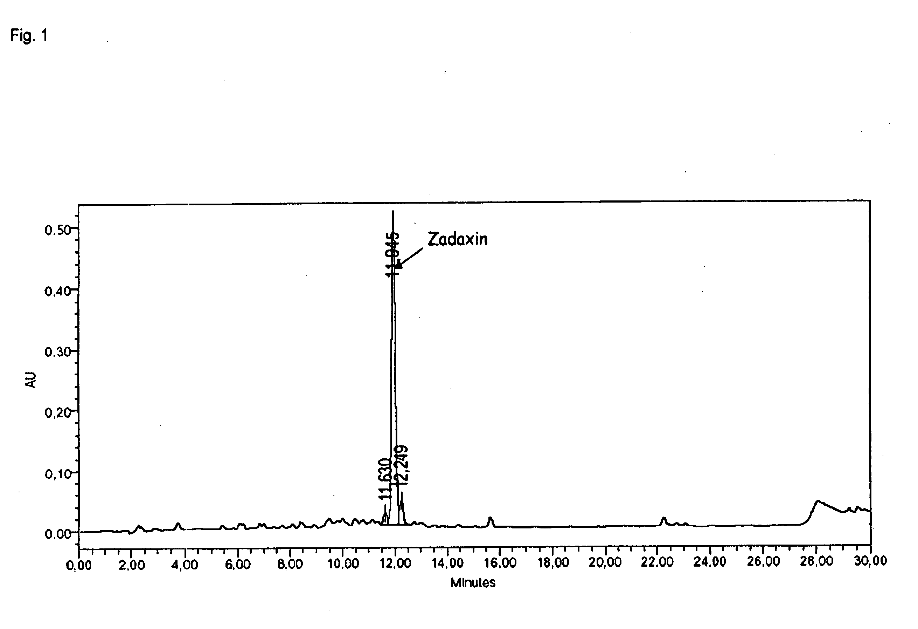

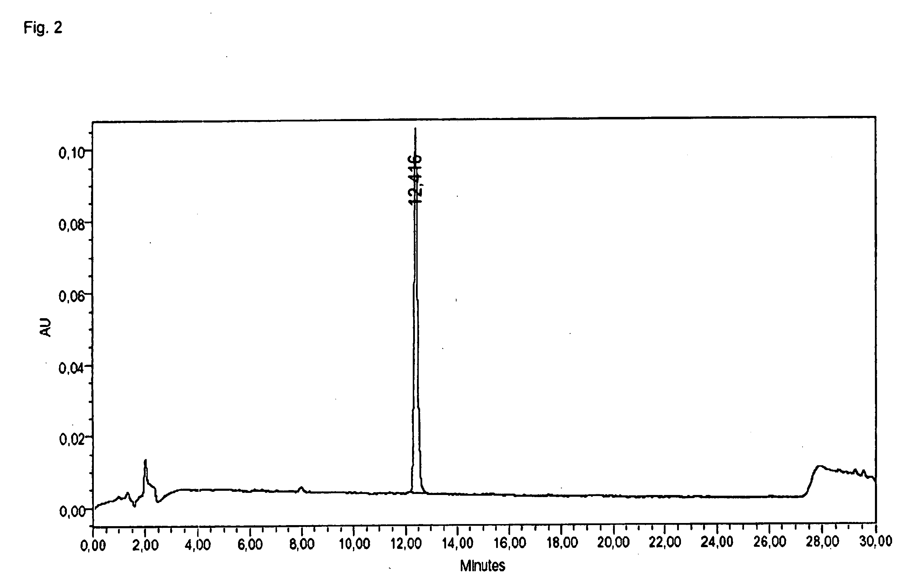

One step cleavage from resin and global deprotection was performed. Portions of 5 g of resin from Step 3 were placed in the glass funnel fitted with a porous disk were treated with cold TFA-H₂O (95:5) (35 mL) for 2 h, then the solution was filtered off and the resin was further washed with TFA-H₂O (95:5) (10 mL). The combined TFA solution was poured on cold *t*butyl methyl ether (350 mL) and the mixture was centrifugated. The solid was isolated by decantation.
MALDI-TOF-MS, calcd. 3106.50. Found: *m*/*z* 3107.36 [M+H]⁺.
RP-HPLC analysis of the crude peptide showed that the product was predominantly pure, amounting to 90% yield (Fig. 1). Two minor contaminating peaks could be easily removed by RP-HPLC on C18 Hypersil with 10-30% acetonitrile water over 45 min., giving pure product with amost complete recovery of the correct product peaki (Fig. 2).

### Example 2: Comparative example

### Ac-Ser-Asp-Ala-Ala-Val-Asp-Thr-Ser-Ser-Glu-Ile-Thr-Thr-Lys-Asp-Leu-Lys-Glu-Lys-Lys-Glu-Val-Val-Glu-Glu-Ala-Glu-Asn-OH (Zadaxin,tymalfasin)

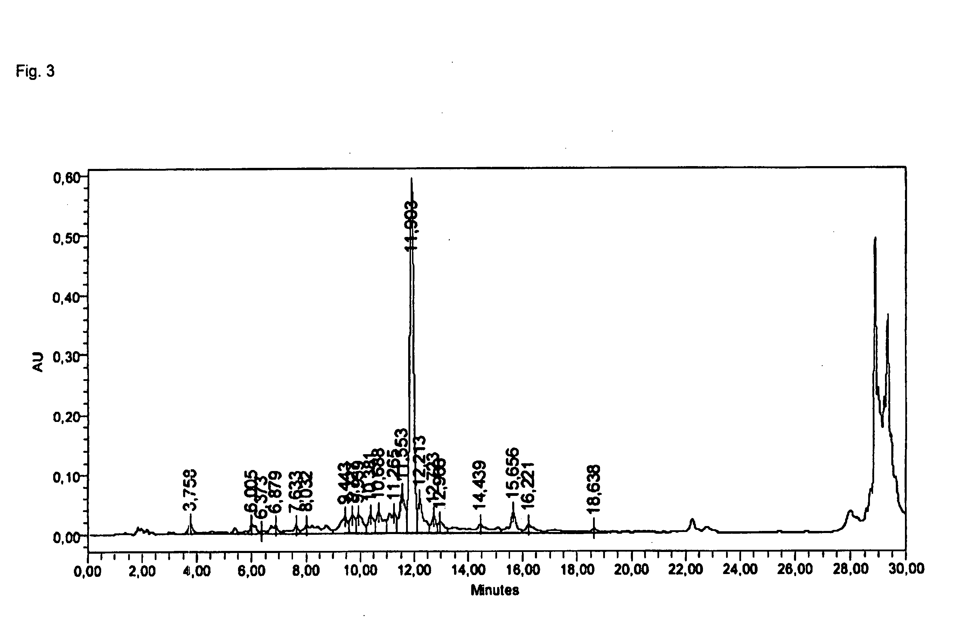

Experimental procedures as described in Examples 1 and 2, except that Fmoc-Sieber-PS-resin (0.59 mmol/g) (3.33 g, 2 mmol) was used instead of Fmoc-Rink-ChemMatrix-resin.
MALDI-TOF-MS, calcd. 3106.50. Found: *m*/*z* 3107.24 [M+H]⁺. The purity and yield of the crude product mix analyzed by RP-HPLC as determined by peak area amounted to 40% yield only (Fig. 3), with a broad spectrum of impurities.

### Example 3: Comparative Example

### Ac-Ser(tBu)-Asp(OtBu)-Als-Ala-Val-Asp(OtBu)-Thr(tBu)-Ser(tBu)-Ser(tBu)-Glu(OtBu)-Ile-Thr(tBu)-Thr(tBu)-Lys(Boe)-Asp(OtBu)-Leu-Lys(Boc)-Glu(OtBu)-Lys(Boc)-Lys(Boc)-Glu(OtBu)-Val-Val-Glu(OtBu)-Glu(OtBu)-Ala-Glu(OtBu)-Asn(Trt)-O-(2-Cl-Trt)-resin

### Step 1

### Ca -terminal anchoring on CTC resin, giving Fmoc-Asn(Trt)-O-TrtCl-resin

2-Cl-TrtCl-resin (0.2 g, 1.64 mmol/g; Ambersynth™ CTC from Rohm & Haas, Paris, France with matrix made from polystyrene-1% divinylbenzene) was placed in a 10 mL polypropylene syringe fitted with a polyethylene filter disk. The resin was then washed with CH₂Cl₂ (5 × 0.5 min), and a solution of Fmoc-Asn(Trt)-OH (0.7 equiv) and DIEA (241 µL) in CH₂Cl₂ (2.5 mL) was added, and the mixture was stirred for 15 min, when extra DIEA (121 µL, total 7 equiv) and the mixture stirred for 45 min. The reaction was terminated by addition of MeOH (160 µL), after a stirring of 10 min. The Fmoc-Asn(Trt)-O-TrtCl-resin was subjected to the following washings with CH₂Cl₂ (3 × 0.5 min) and DMF (3 × 0.5 min). The loading calculated by Fmoc determination was 0.8 mmol/g.

### Step 2

### H-Ser(tBu)-Asp(OtBu)-Ala-Ala-Val-Asp(OtBu)-Thr(tBu)-Ser(tBu)-Ser(tBu)-Glu(OtBu)-Ile-Thr(tBu)-Thr(tBu)-Lys(Boc)-Asp(OtBu)-Leu-Lys(Boc)-Gim(OtBu)-Lys(Boc)-Lys(Boc)-Glm(OtBu)-Val-Val-Glu(OtBu)-Glu(OtBu)-Ala-Glu(OtBu)-Asn(Trt)-ClTrt-PS-resin

The Fmoc group was removed and Fmoc-aa-OHs (10 equiv) were sequentially added to the above peptidyl-resin (step 1) together with DIEA (20 equiv) in DMF (9 mL), by HCTU (9.6 equiv) in DMF an ABI automatic synthesizer 433A with 60 min coupling time.

### Step 3

### Ac-Ser(tBu)-Asp(OtBu)-A/a-Ala-Val-Asp(OtBu)-Thr(tBu)-Ser(tBu)-Ser(tBu)-Glu(OtBu)-Ile-Thr(tBu)-Thr(tBu)-Lys(Boc)-Asp(OtBu)-Leu-Lys(Boc)-Glu(OtBu)-Lys(Boc)-Lys(Boc)-Glu(OtBu)-Val-Val-Glu(OtBu)-Glu(OtBu)-Ala-Glu(OtBu) Asn(Trt)-ClTrt-PS-resin

The final acetylation was carried out with Ac₂O (0.5 mmol) and DIEA (5 mmol) in DMF (2 mL) for 15 min with sporadic manual stirring, where ninhydrin was negative.

### Step 4

### Ac-Ser(tBu)-Asp(OtBu)-Ala-Ala-Val-Asp(OtBu)-Thr(tBu)-Ser(tBu)-Ser(tBu)-Glu(OtBu)-Ile-Thr(tBu)-Thr(tBu)-Lys(Boc)-Asp(OtBu)-Leu-Lys(Boc)-Glu(OtBu)-Lys(Sec)-Lys(Boc)-Glu(OtBu)-Val-Val-Glu(OtBu)-Glu(OtBu)-Ala-Glu(OtBu)-Asn(Trt)-OH

The protected peptide from step 3 above was cleaved from the resin by TFA-Et₃SiH-CH₂Cl₂ (1:1:98) (5 × 30 sec). Filtrate was collected on H₂O (4 mL) and the H₂O was partially removed under reduced pressure. Acetonitrile (ACN) was then added to dissolve solid that appeared during the H₂O removal, and the solution was lyophilized.

### Step 5

### Ac-Ser-Asp-Ala-Ala-Val-Asp-Thr-Ser-Ser-Glu-Ile-Thr-Thr-Lys-Asp-Leu-Lys-Glu-Lys-Lys-Glu-Val-Val-Glu-Glu-Ala-Glu-Asn-OH (Zadaxin,tymalfasin)

The protected peptide from Step 4 was dissolved in TFA-H₂O (95:5, 5 mL) and the mixture was allowed to stir for 1 h. Then the solution was filtered off and the resin was further washed with TFA-H₂O (95:5) (1 mL). The combined TFA solution was poured on cold *t*butyl methyl ether (25 mL) and the mixture was centrifugated. The solid was isolated by decantation. Then, H₂O (5 mL) was added and lyophilized.
MALDI-TOF-MS, calcd. 3106.50. Found: *m*/*z* [M+H]⁺ 3107.98.

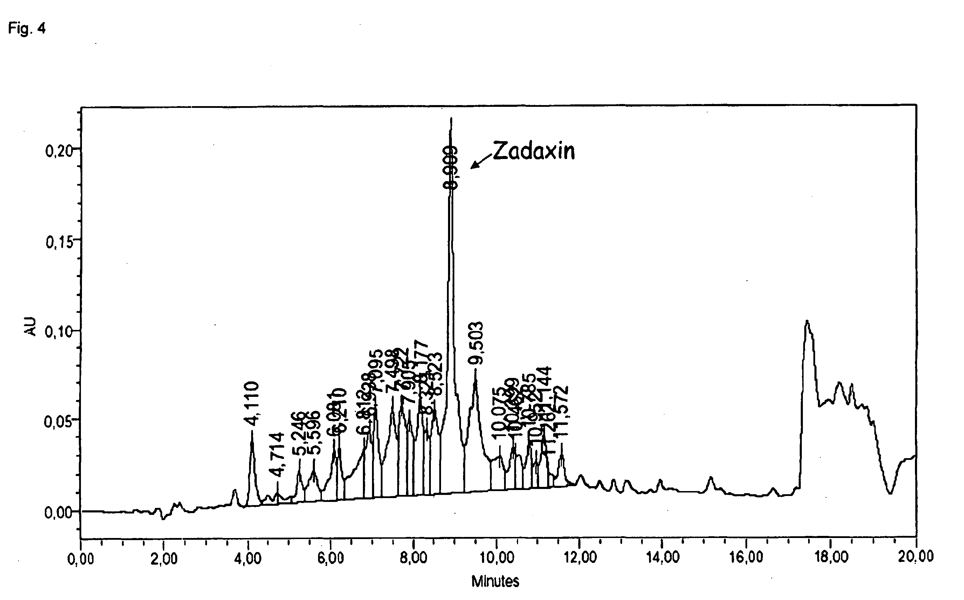

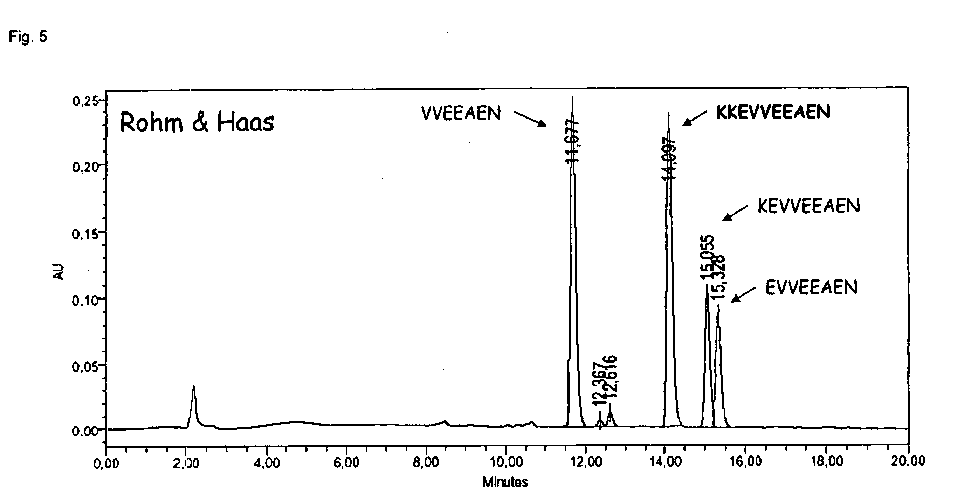

The crude purity and yield, as determined by RP-HPLC peak area, amounted to disappointing 20% proper product with a strong background smear of multiple improper chain variants (Fig. 4). - Careful repetition of the solid-phase synthesis of a shortened fragment (Lys19 up to Asn28) with manual coupling and ninhydrin testing after every step showed that chain synthesis often effectively terminated after incorporation of V22; three consecutive couplings E21, K20 and K 19 proved to be highly inefficient, requiring capping of unreacted chains. Double coupling, elongated coupling times and using more eq. of Fmoc amino acid did not satisfactorily overcome the problem, not allowing to establish significantly more efficient synthesis this way (Fig. 5).

### Example 4: Comparative example

### Ac-Ser-Asp-Ala-Ala-Val-Asp-Thr-Ser-Ser-Glu-Ile-Thr-Thr-Lys-Asp-Leu-Lys-Glu-Lys-Lys-Glu-Val-Val-Glu-Glu-Ala-Glu-Asn-OH (Zadaxin, tymalfasin)

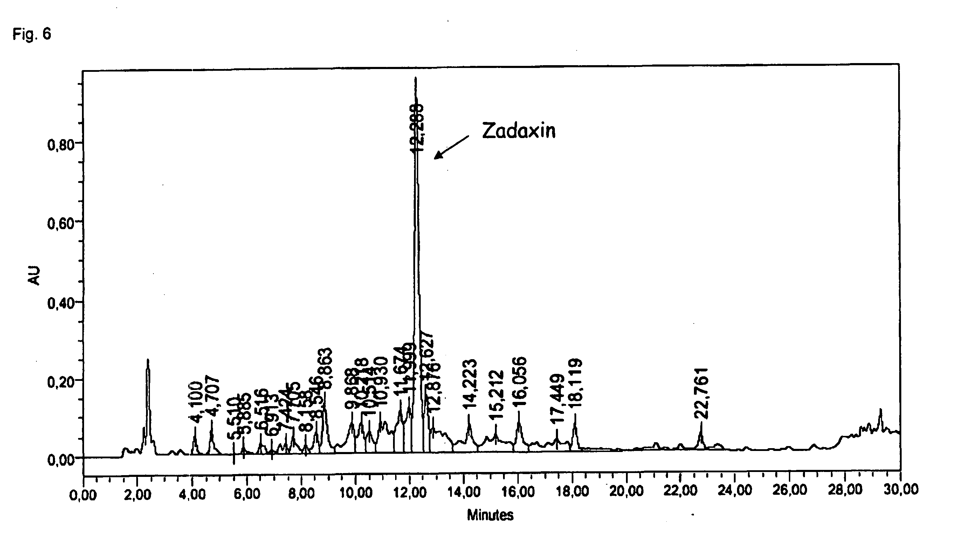

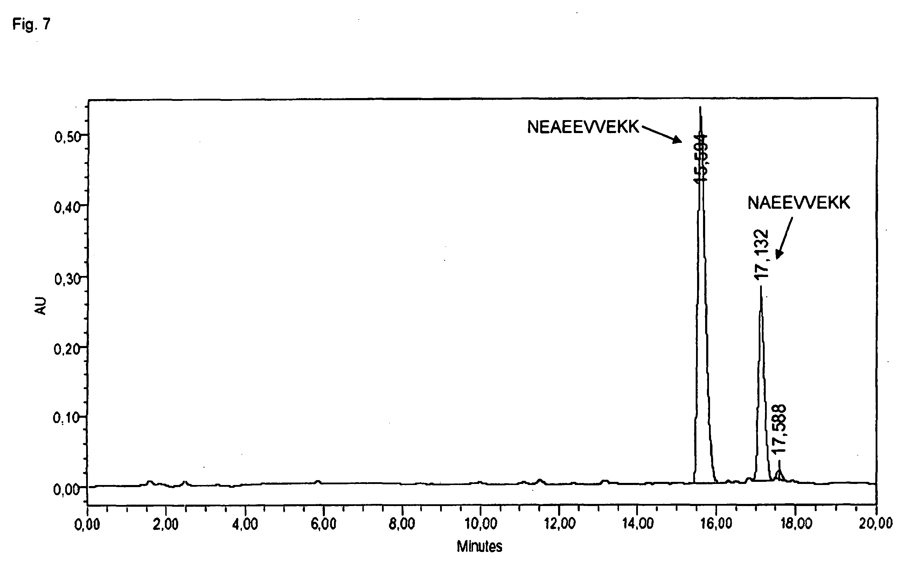

Experimental procedures essentially as described in Example 2, except that Fmoc-Rink handle was incorporated onto MBHA resin (4-methylbenzhydrylamine resin LL on polystyrene-1% divinylbenzene basis, Novabiochem™, Merck/Germany), Fmoc-Asp-OtBu was side-chain anchored as described in Step1, Example I onto Rink aminomethyl grafted linker after removal of Fmoc group of the Rink moiety, loading was determined to amount to 0.66 mmol/g. Couplings were carried out with HCTU (10 eq.) for 35 min. with double couplings at positions D28 (i.e.N28), E27,E21,K20,K19. One step cleavage and deprotection was carried out with TFA-H2O (95:5) after acetylation has been carried out as described above. The crude purity and yield obtained, as determined by RP-HPLC, was 27% (Fig. 6).

### Example 5

### NH2-Lys-Lys-Glu-Val-Val-Glu-Glu-Ala-Glu-Asn-OH (fragment 19-28 of tymalfasin)

Side chain anchoring and synthesis was performed on Rink-Chemmatrix resin essentially as described in example 1, except that no double coupling was performed at the sequence positions indicated in example 1 but a uniform, single coupling time of 35 min. was applied. Deletion of the Glu residue No. 27 was observed as the only but dominant chain termination byproduct in the HPLC diagram (Fi.g 7). -As set forth in example 1, this byproduct could be successfully suppressed by extended, repeated coupling at E27.

### Example 6

### NH2-Lys-Lys-Glu-Val-Val-Glu-Glu-Ala-Glu-Asn-OH (fragment 19-28 of tymalfasin)

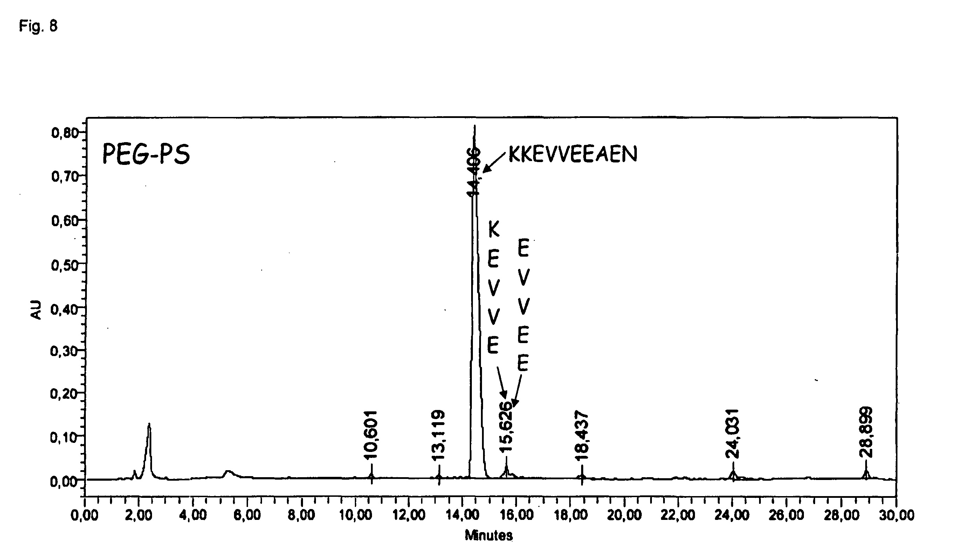

Side chain anchoring and synthesis was performed essentially as described in example 1, except that a PEG-MBHA resin from Millipore-Waters (MeO[PEG]2000-CO-Om-MBHA-PS-resin, similiarly described in Kates et al., High-load PEG-polystyrene graft supports for solid-phase synthesis, supra) that was grafted with a Rink aminomethyl linker was employed. The loading of the Rink-PEG-MBHA resin was determined with 0.55 mmol/g. Specially, double couplings were performed at the sequence positions E27,E21,K20,K19. The crude purity and yield determined by RP-HPLC amounted to 92% (Fig. 8).

## Claims

1. Peptide conjugate of formula I 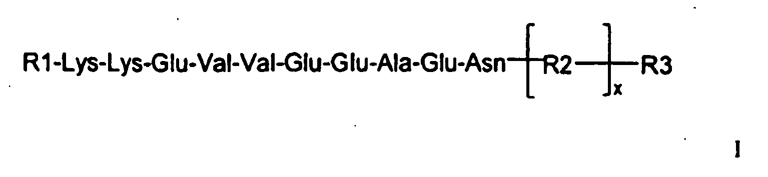 wherein each amino acid residue is individually protected or unprotected, R3 is a PEG resin solid phase which is comprising an integral linker, R2 is a grafted linker with x being 0 or 1 and R1 is hydrogen, a protecting group or a peptidyl radical, preferably a peptidyl radical counting less than 50, preferably less than 25 amino acid residues, and
wherein, if R1 is a peptidyl radical, the individual amino acid residues of said radical are individually protected or unprotected and the N-terminus of said radical is free, is acetylated or is protected with a protecting group Y.

2. Peptide conjugate according to the preceding claim, **characterised in that** the protecting group Y is an Fmoc group, a Dde-type group, a Boc group or a derivative of said protecting groups, preferably a derivative of a Fmoc or Dde-type group, that comprises a moiety that allows of reversible affinity binding of the thus protected peptide to a suitable chromatography solid matrix material.

3. Peptide conjugate according to claim 1, **characterised in that** the protecting group Y allows of reversible affinity binding to an immobilised-metal affinity chromatography solid matrix material.

4. Peptide conjugate according to one of the preceding claims, **characterised in that** R1 is Y-Ser-Asp-Ala-Ala-Val-Asp-Thr-Ser-Ser-Glu-Ile-Thr-Thr-Lys-Asp- Leu-Lys-Glu-, Ac-Ser-Asp-Ala-Ala-Val-Asp-Thr-Ser-Ser-Glu-Ile-Thr-Thr-Lys-Asp- Leu-Lys-Glu or H- Ser-Asp-Ala-Ala-Val-Asp-Thr-Ser-Ser-Glu-Ile-Thr-Thr-Lys-Asp- Leu-Lys-Glu-Leu-Lys-Glu with Y having the meaning named before.

5. Method of synthesizing Thymosin α₁, a C-terminally truncated version of Thymosin α₁ comprising residues 1-27, an N-terminally truncated version of Thymosin α₁ comprising at least residues 19-28 or a truncated version comprising at least residues 19-27 of mature Thymosin α₁ on a solid phase comprising the steps of
a. coupling a suitably protected FMOC-Glu, FMOC-Asp or FMOC-Asn residue onto a PEG resin solid phase which PEG resin is preferably selected from the group comprising polystyrene-PEG mixed resins, PEG polyether-polyester mixed resins or PEG polyether-polyamide mixed resins and essentially pure PEG resins
b. elongating the peptide chain by FMOC synthesis wherein the side-chains of individual amino acids may be derivatized with suitable protecting groups
c. optionally acetylating the last, N-terminal residue and
d. cleaving the peptide from the resin

6. Method according to claim 5, **characterised in that** the peptide synthesized is Thymosin a, having the sequence
1-Ser-Asp-Ala-Ala-Val-Asp-Thr-Sar-Ser-Glu-Ile-Thr-Thr-Lys-Asp-Leu-Lys-Glu-Lys-Lys-Glu-Val-Val-Glu-Glu-Ala-Glu-Asn-28
wherein the individual amino acid chains are unprotected or are suitably protected.

7. Method according to claim 5, **characterised in that** the PEG resin is an amphiphilic resin.

8. Method according to claim 7, **characterised in that** the loading capacity of the PEG resin is at least 0.5 mmol/g and which PEG resin preferably is essentially devoid of polystyrene co-polymeric share.

9. Method according to claim 7, **characterised in that** the PEG-resin with the exception of the integral and/or grafted linkers is a substantially pure polyether-PEG resin, preferably that it is a pure PEG-polyether resin.

## Patentansprüche

1. Peptidkonjugat der Formel I 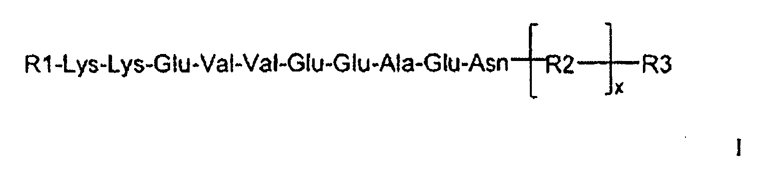 wobei die einzelnen Aminosäurereste jeweils individuell geschützt oder ungeschützt vorliegen, R3 eine PEG-Harzfestphase ist, die einen integralen Linker umfasst, R2 ein aufgepfropfter Linker mit x gleich 0 oder 1 ist, und R1 Wasserstoff, eine Schutzgruppe oder ein Peptidylrest ist, vorzugsweise ein Peptidylrest ist, der weniger als 50, vorzugsweise weniger als 25 Aminosäurereste zählt, und wobei, falls R1 ein Peptidylrest ist, die einzelnen Aminosäurereste des besagten Rests individuell geschützt oder ungeschützt vorliegen und der N-Terminus des besagten Rests in freier, acetylierter oder mit einer Schutzgruppe Y geschützter Form vorliegt.

2. Peptidkonjugat nach dem vorhergehenden Anspruch, **dadurch gekennzeichnet, dass** es sich bei der Schutzgruppe Y um eine Fmoc-Gruppe, eine Gruppe vom Dde-Typ, eine Boc-Gruppe oder ein Derivat dieser Schutzgruppen, vorzugsweise ein Derivat einer Fmoc- oder Dde-Typ-Gruppe, handelt, die eine Gruppierung umfasst, die die reversible Affinitätsbindung des so geschützten Peptids an ein geeignetes chromatographisches Feststoffmatrixmaterial gestattet.

3. Peptidkonjugat nach Anspruch 1, **dadurch gekennzeichnet, dass** die Schutzgruppe Y die reversible Affinitätsbindung an ein affinitätschromatographisches Feststoffmatrixmaterial mit immobilisiertem Metall gestattet.

4. Peptidkonjugat nach einem der vorhergehenden Ansprüche, **dadurch gekennzeichnet, dass** R1 Y-Ser-Asp-Ala-Ala-Val-Asp-Thr-Ser-Ser-Glu-Ile-Thr-Thr-Lys-Asp-Leu-Lys-Glu-, Ac-Ser-Asp-Ala-Ala-Val-Asp-Thr-Ser-Ser-Glu-Ile-Thr-Thr-Lys-Asp-Leu-Lys-Glu oder H-Ser-Asp-Ala-Ala-Val-Asp-Thr-Ser-Ser-Glu-Ile-Thr-Thr-Lys-Asp-Leu-Lys-Glu-Leu-Lys-Glu ist, wobei Y die zuvor genannte Bedeutung aufweist.

5. Verfahren zur Synthese von Thymosin α₁, einer C-terminal verkürzten Version von Thymosin α₁, die die Reste 1-27 umfasst, einer N-terminal verkürzten Version von Thymosin α₁, die mindestens die Reste 19-28 umfasst, oder einer verkürzten Version, die wenigstens die Reste 19-27 des reifen Thymosin α₁ umfasst, an einer Festphase umfassend die Schritte
a. Koppeln eines geeignet geschützten FMOC-Glu-, FMOC-Asp- oder FMOC-Asn-Rests an eine PEG-Harzfestphase, wobei das PEG-Harz vorzugsweise ausgewählt ist aus der Gruppe bestehend aus Polystyrol-PEG-Mischharzen, PEG-Polyether-Polyester-Mischharzen oder PEG-Polyether-Polyamid-Mischharzen und im Wesentlichen reinen PEG-Harzen,
b. Verlängern der Peptidkette mittels FMOC-Synthese, wobei die Seitenketten individueller Aminosäuren mit geeigneten Schutzgruppen derivatisiert sein können,
c. wahlweises Acetylieren des letzten, N-terminalen Rests und
d. Abspalten des Peptids vom Harz.

6. Verfahren nach Anspruch 5, **dadurch gekennzeichnet, dass** es sich bei dem synthetisierten Peptid um Thymosin α₁ mit der Sequenz
1-Ser-Asp-Ala-Ala-Val-Asp-Thr-Ser-Ser-Glu-Ile-Thr-Thr-Lys-Asp-Leu-Lys-Glu-Lys-Lys-Glu-Val-Val-Glu-Glu-Ala-Glu-Asn-28
handelt, wobei die einzelnen Aminosäureketten ungeschützt oder in geeigneter Weise geschützt vorliegen.

7. Verfahren nach Anspruch 5, **dadurch gekennzeichnet, dass** es sich bei dem PEG-Harz um ein amphiphiles Harz handelt.

8. Verfahren nach Anspruch 7, **dadurch gekennzeichnet, dass** die Beladungskapazität des PEG-Harzes mindestens 0,5 mmol/g beträgt und wobei das PEG-Harz vorzugsweise im Wesentlichen keinen Polystyrol-Copolymeranteil aufweist.

9. Verfahren nach Anspruch 7, **dadurch gekennzeichnet, dass** es sich bei dem PEG-Harz mit Ausnahme der integralen und/oder aufgepfropften Linker um ein weitgehend reines Polyether-PEG-Harz handelt, vorzugsweise, dass es sich um ein reines PEG-Polyether-Harz handelt.

## Revendications

1. Conjugué peptidique de formule 1 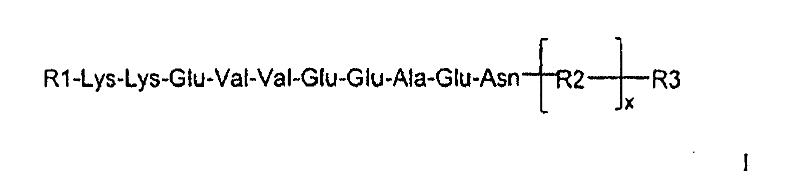 dans laquelle chaque résidu d'acide aminé est individuellement protégé ou non protégé, R3 est une phase solide en résine PEG qui comprend un lieur intégral, R2 est un lieur greffé, x valant 0 ou 1, et R1 est hydrogène, un groupe protecteur ou un radical peptidyle, de préférence un radical peptidyle comptant moins de 50, de préférence moins de 25 résidus d'acides aminés, et dans laquelle, si R1 est un radical peptidyle, les résidus d'acides aminés individuels dudit radical sont individuellement protégés ou non protégés et l'extrémité N-terminale dudit radical est libre, est acétylée ou est protégée par un groupe protecteur Y.

2. Conjugué peptidique selon la revendication précédente, **caractérisé en ce que** le groupe protecteur Y est un groupe Fmoc, un groupe de type Dde, un groupe Boc ou un dérivé desdits groupes protecteurs, de préférence un dérivé d'un groupe Fmoc ou de type Dde, qui comprend un fragment qui permet la liaison d'affinité réversible du peptide ainsi protégé à un matériel de matrice solide de chromatographie approprié.

3. Conjugué peptidique selon la revendication 1, **caractérisé en ce que** le groupe protecteur Y permet une liaison d'affinité réversible à un matériel de matrice solide de chromatographie d'affinité métallique immobilisé.

4. Conjugué peptidique selon l'une quelconque des revendications précédentes, **caractérisé en ce que** R1 est Y-Ser-Asp-Ala-Ala-Val-Asp-Thr-Ser-Ser-Glu-Ile-Thr-Thr-Lys-Asp-Leu-Lys-Glu-, Ac-Ser-Asp-Ala-Ala-Val-Asp-Thr-Ser-Ser-Glu-Ile-Thr-Thr-Lys-Asp-Leu-Lys-Glu ou H-Ser-Asp-Ala-Ala-Val-Asp-Thr-Ser-Ser-Glu-Ile-Thr-Thr-Lys-Asp-Leu-Lys-Glu-Leu-Lys-Glu, Y ayant la signification indiquée ci-avant.

5. Méthode de synthèse de thymosine α₁, d'une version tronquée à l'extrémité C-terminale de la thymosine α₁ comprenant les résidus 1 à 27, d'une version tronquée à l'extrémité N-terminale de la thymosine α₁ comprenant au moins les résidus 19 à 28 ou d'une version tronquée comprenant au moins les résidus 19 à 27 de la thymosine α₁ mature sur une phase solide, comprenant les étapes consistant à :
a. coupler un résidu FMOC-Glu, FMOC-Asp ou FMOC-Asn protégé de façon appropriée à une phase solide en résine PEG, ladite résine PEG étant de préférence choisie dans le groupe comprenant les résines mixtes polystyrène-PEG, les résines mixtes PEG polyéther-polyester ou les résines mixtes PEG polyéther-polyamide et les résines PEG essentiellement pures
b. allonger la chaîne peptidique par synthèse FMOC, les chaînes latérales des acides aminés individuels pouvant être dérivatisées avec des groupes protecteurs appropriés
c. acétyler éventuellement le dernier résidu N-terminal, et
d. cliver le peptide de la résine.

6. Méthode selon la revendication 5, **caractérisée en ce que** le peptide synthétisé est la thymosine α₁ ayant la séquence
1-Ser-Asp-Ala-Ala-Val-Asp-Thr-Ser-Ser-Glu-Ile-Thr-Thr-Lys-Asp-Leu-Lys-Glu-Lys-Lys-Glu-Val-Val-Glu-Glu-Ala-Glu-Asn-28
dans laquelle les chaînes d'acides aminés individuelles sont non protégées ou sont protégées de façon appropriée.

7. Méthode selon la revendication 5, **caractérisée en ce que** la résine PEG est une résine amphiphile.

8. Méthode selon la revendication 7, **caractérisée en ce que** la capacité de charge de la résine PEG est au moins égale à 0,5 mmol/g et **en ce que** ladite résine PEG est de préférence essentiellement exempte de la partie polystyrène copolymère.

9. Méthode selon la revendication 7, **caractérisée en ce que** la résine PEG, à l'exception des lieurs intégraux et/ou greffés, est une résine polyéther-PEG sensiblement pure, de préférence **en ce qu'**il s'agit d'une résine PEG-polyéther pure.
